# EUROPEAN PATENT APPLICATION

(11) **EP 4 825 962 A1**
(43) Date of publication of application: **21.10.2026**
(21) Application number: 25171089.3
(22) Date of filing: 16.04.2025
(51) Int. Cl.: C12N 9/10, C12N 9/80, C12N 9/88, C12P 19/26

(54) **CHITOSAN SYNTHASES AND USES THEREOF**

(71) Applicant: Universität Münster Körperschaft des öffentlichen Rechts, 48149 Münster (DE)
(72) Inventor: Moerschbacher, Bruno, 48161 Münster (DE); Cord-Landwehr, Stefan, 48149 Münster (DE); Singh, Ratna, 48149 Münster (DE)
(74) Representative: Maiwald GmbH

(57) **Abstract**

The present invention relates to isolated polypeptides comprising the amino acid sequence of SEQ ID NO:1, SEQ ID NO:2 and/or SEQ ID NO:3 or a variant thereof wherein said polypeptides preferably have chitinosanase, chitin deacetylase and/or chitin synthase activity, as well as nucleic acids encoding them, host cells comprising such nucleic acids and methods of use and uses of these polypeptides, nucleic acids and host cells.

## Description

### Filed of the invention

The present invention lies in the field of enzymes and recombinant chitosan production and relates to novel multi-domain chitosan synthases and their individual domains and variants thereof, nucleic acids encoding them, host cells comprising such nucleic acids as well as methods of use and uses thereof.

### Background

Chitosans are rare in nature and are therefore industrially produced mostly by partial alkaline deacetylation of chitin, which is predominantly extracted from shrimp and crab waste. The deacetylation of chitin results in a mixture of raw chitosans; these structurally and functionally poorly characterized mixtures still dominate the market today.

By carefully controlling the deacetylation conditions, two of the three parameters that structurally characterize chitosans can be controlled within certain limits: the degree of polymerization (DP) and the fraction of acetylation (FA). The chitosans produced in this way are currently used to investigate structure/function relationships of chitosans, e.g., regarding their antimicrobial, plant-strengthening, or medical activities.

The third structural parameter, the pattern of acetylation (PA), cannot be chemically controlled: all chitosans produced according to the above described methods have a roughly random distribution of acetyl groups in the polymer.

It is presently hypothesized that in nature, chitosans are also synthesized via chitin in a two-step process. Unlike the almost ubiquitously occurring chitin, chitosan is rather rare in nature; it is found almost exclusively in a subgroup of zygomycetes, the *Mucoromycetes.* In all chitin-containing organisms, a transmembrane chitin synthase (CHS) polymerizes the N-acetylglucosamine (GlcNAc) monomers into fully acetylated chitin, which is successively released through a tunnel to the outside, where the chitin polymers align and, probably supported by interaction with specific chitin-binding proteins, crystallize into chitin fibrils. In *Mucoromycetes* fungi, the chitin chains emerging from the tunnel into the extracellular space are assumed to be converted into chitosans in a second step and before fibril formation by membrane-associated chitin deacetylases (CDA), since once the chitin chains have crystallized into chitin fibrils, they can no longer be deacetylated by CDA. An enzymatic conversion of chitin to chitosan *in vitro* has therefore not been successful despite numerous attempts.

The chemical processes for extracting chitin and especially for converting it into chitosans are problematic. They are energy- and chemical-intensive, require large volumes of clean water, and generate massive quantities of salt- and protein-containing wastewater. Although biotechnological methods for extracting chitin exist, these are not commercially competitive. More importantly, regardless of its source and extraction method, chitin is always crystalline and thus unsuitable as a substrate for biotechnological conversion to chitosans using chitin deacetylases (CDAs), since all known enzymes can only deacetylate the chitin chain directly emerging from the chitin synthase, i.e. before it crystallizes into chitin fibrils. Consequently, a true biotechnological production of chitosan must utilize a natural or genetically engineered chitosan-producing organism.

Currently, only Mucorales fungi are suitable as natural producers. However, in fungal cell walls, chitin and, in this specific case, chitosan are covalently linked with other cell wall polymers, particularly beta-glucans. This makes extraction difficult and results in lower purity compared to conventionally (chemically) produced chitosans. Additionally, fungal chitin and the resulting fungal chitosan have relatively low molecular weights (DP values around 200-400) compared to chitin from crab and shrimp shells (DP 1,000-3,000) or squid skeletons (DP 5,000-10,000). While reducing DP through partial acidic or enzymatic hydrolysis is routinely done, increasing DP is not possible (except through artificial crosslinking, which does not yield pure chitin or chitosan). A high DP is therefore an important quality criterion for chitosans.

DP, FA, and PA all have significant influence on structure and thus in turn on function. This is important because even the best conventionally chemically produced chitosans can only be controlled and defined in terms of DP and FA, while PA remains random due to the chemical process of alkaline deacetylation used. However, since PA also significantly influences bioactivities, its control is essential for the further development of reliable and efficient chitosan-based products. The production of such chitosans with defined PA is currently only established on a laboratory scale and due to the existing drawbacks, scaling to larger scales would definitely not be competitive with current conventional methods.

There thus exists need for novel strategies that overcome existing issues in the production of high quality chitosans and allow the recombinant production of structurally fully defined chitosans at larger scales.

### Summary of the Invention

The present invention is based on the identification of a novel three-domain enzyme which combines a chitin synthase domain with a chitin deacetylase domain and a chitinosanase domain to produce structurally defined chitosans. This particular enzyme and its three functionalities that seamlessly function together to produce chitosans provides for a promising means to overcome existing challenges and allow recombinant production of chitosans in suitable host organisms.

In a first aspect, the present invention is thus directed to an isolated polypeptide comprising an amino acid sequence that has at least 80 % sequence identity to the amino acid sequence set forth in SEQ ID NO:1. Said polypeptide preferably has chitinase activity, more preferably chitinosanase activity.

In various embodiments, its chitinase activity, preferably chitinosanase activity, is under identical conditions at least 80 % relative to the chitinosanase activity of a polypeptide consisting of the full length sequence of SEQ ID NO:4.

In various embodiments, said polypeptide recognizes and cleaves a partially deacetylated chitin or chitosan polymer at the motif AA | D, wherein A represents N-acetyl-D-glucosamine (GlcNAc) and D represents D-glucosamine (GlcN).

In various embodiments, the polypeptide does not comprise the full length sequence of SEQ ID NO:4.

The polypeptide may be up to 1000 amino acids in length, preferably up to 900, up to 800, up to 700, up to 600, up to 500, up to 400 or up to 300 amino acids in length. The polypeptide's length may be at least 250 amino acids in length, preferably at least 270, more preferably at least 290. In various embodiments, in said polypeptides according to the first aspect, any one or both, preferably both, of the amino acid residues D at position 111 and E at position 113, using the positional numbering of SEQ ID NO:1, are invariable.

In a second aspect, the present invention is directed to an isolated polypeptide comprising an amino acid sequence that has at least 80 % sequence identity to the amino acid sequence set forth in SEQ ID NO:2. Said polypeptide preferably has chitin deacetylase activity. The chitin deacetylase activity may, under identical conditions, be at least 80 % relative to the chitin deacetylase activity of a polypeptide consisting of the full length sequence of SEQ ID NO:4.

In various embodiments, said polypeptide does not comprise the full length sequence of SEQ ID NO:4.

The polypeptide of the second aspect may have a length of up to 1000 amino acids, preferably up to 900, up to 800, up to 700, up to 600, up to 500, up to 400, up to 300, up to 250, or up to 200 amino acids. The minimum length may, in various embodiments, be at least 180 amino acids in length, preferably at least 190, more preferably at least 195 amino acids. In various embodiments, in the isolated polypeptide of the second aspect, any one or more, preferably all, of the amino acid residues D at position 6, D at position 7, H at position 57, H at position 61, and H at position 164, using the positional numbering of SEQ ID NO:2, are invariable. In various embodiments, any one or more, preferably all, of the amino acid residues R at position 95, Y at position 98, D at position 129, D at position 132, and W at position 133, using the positional numbering of SEQ ID NO:2, are invariable. These two lists are freely combinable. It is however preferred that all of the indicated positions are invariable.

In a third aspect, the present invention is directed to an isolated polypeptide comprising an amino acid sequence that has at least 80 % sequence identity to the amino acid sequence set forth in SEQ ID NO:3. In various embodiments, the polypeptide has chitin synthase activity. The chitin synthase activity may, under identical conditions, be at least 80 % relative to the chitin synthase activity of a polypeptide consisting of the full length sequence of SEQ ID NO:4.

In various embodiments, the polypeptide does not comprise the full length sequence of SEQ ID NO:4.

The isolated polypeptide of the third aspect may be up to 1000 amino acids in length, preferably up to 900, up to 800, up to 700, up to 600, up to 500, up to 400, up to 300, or up to 250 amino acids in length. Its minimum length is, in various embodiments, at least 200 amino acids, preferably at least 210, more preferably at least 220 amino acids. In the isolated polypeptide of the third aspect, preferably any one or more, more preferably all, of the amino acid residues D at position 88, D at position 90, D at position 180, R at position 219, and W at position 220, using the positional numbering of SEQ ID NO:3, are invariable.

In another aspect, the present invention also relates to an isolated polypeptide comprising any two or three of
(1) an amino acid sequence that has at least 80 % sequence identity to the amino acid sequence set forth in SEQ ID NO:1;
(2) an amino acid sequence that has at least 80 % sequence identity to the amino acid sequence set forth in SEQ ID NO:2; and
(3) an amino acid sequence that has at least 80 % sequence identity to the amino acid sequence set forth in SEQ ID NO:3.

In various embodiments, such an isolated polypeptide may comprise (1) and (2) but not (3). In such embodiments, the polypeptide preferably has chitinosanase and chitin deacetylase activity as defined above for the polypeptides of the first and second aspect of the invention.

In various embodiments, such an isolated polypeptide may comprise (2) and (3) but not (1). In such embodiments, the polypeptide preferably has chitin synthase and chitin deacetylase activity as defined above for the polypeptides of the second and third aspect of the invention.

In various embodiments, such an isolated polypeptide may comprise (1) and (3) but not (2). In such embodiments, the polypeptide preferably has chitin synthase and chitinosanase activity as defined above for the polypeptides of the first and third aspect of the invention.

In various embodiments, the isolated polypeptide comprises the amino acid sequence of any one of SEQ ID Nos. 5-7 or a variant thereof that has at least 80 % sequence identity to any one of SEQ ID Nos. 5-7.

In another aspect, the present invention also includes a nucleic acid molecule encoding the polypeptide of any one of the other aspects of the invention. The nucleic acid molecule may be a plasmid or vector and may comprise other elements.

In a still further aspect, the invention is also directed to a host cell comprising the nucleic acid molecule according to the invention. Said host cell may be a prokaryotic cell, preferably a bacterial cell, such as *E. coli.*

In still another aspect, the invention is directed to a method for the recombinant production of chitosans comprising cultivating the host cell according as described herein, wherein the host cell comprises a nucleic acid molecule encoding the polypeptide according to the invention, wherein the polypeptide comprises an amino acid sequence that has at least 80 % sequence identity to any one or both of SEQ ID Nos. 1 and 2, under conditions that allow the functional expression of the encoded polypeptide.

In various embodiments of the method, the chitosan is secreted into the extracellular space and the method optionally comprises the step of isolating the chitosan from the culture medium.

In a still further aspect, the present invention features a method for the recombinant production of chitin comprising cultivating the host cell according to the invention, wherein the host cell comprises a nucleic acid molecule encoding the polypeptide according to the invention, wherein the polypeptide comprises an amino acid sequence that has at least 80 % sequence identity to SEQ ID NO:3, under conditions that allow the functional expression of the encoded polypeptide.

Also encompassed by the invention is the use of the isolated polypeptides according to the invention, wherein the polypeptide comprises an amino acid sequence that has at least 80 % sequence identity to any one or both of SEQ ID Nos. 1 and 2, for the recombinant production of chitosans.

Alternatively or additionally, the use may be of the isolated polypeptide according to the invention, wherein the polypeptide comprises an amino acid sequence that has at least 80 % sequence identity to SEQ ID NO:3, for the recombinant production of chitin.

In a still further aspect, the invention is directed to a chitosan molecule or a mixture of chitosan molecules produced by the isolated polypeptide according to the invention, the host cell according to the invention or the method according to the invention, wherein the polypeptide comprises an amino acid sequence that has at least 80 % sequence identity to the amino acid sequence set forth in SEQ ID NO:1.

### Brief description of the figures

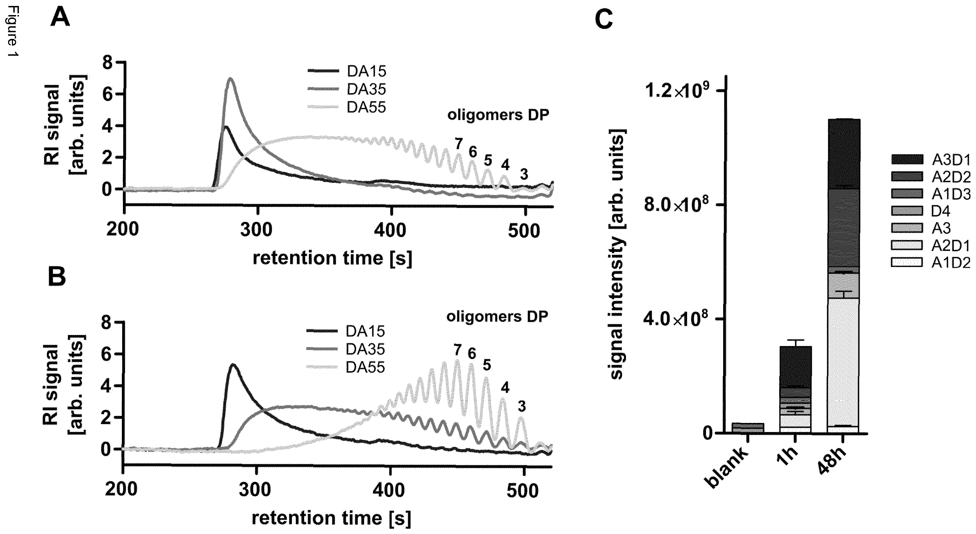

Figure 1 shows SEC-RI chromatograms of chitosan polymers with DA15, DA35, and DA55 after 1 h (A) and 48 h (B) incubation with the chitinosanase domain. The peaks corresponding to oligomers of DP 3-7 are labeled for DA55. (C) Profiles of oligomer products of DP 3 and DP 4 released from DA55 substrate after 1 h or 48 h digestion with the chitinosanase domain (blank: 48 h digestion without addition of enzyme).

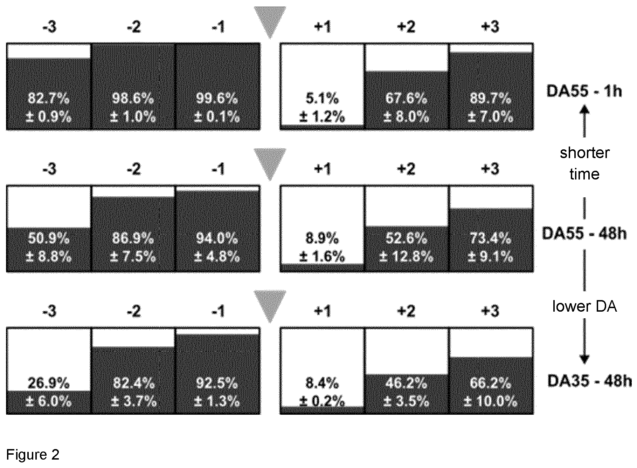

Figure 2 shows the subsite preferences of the chitinosanase domain. The squares are divided according to the percentage of the relative abundances (molar fractions) of GlcN (D, white) and GlcNAc (A, blue) at the subsite, left (-3), (-2) and (-1) and right (+1), (+2) and (+3) of the catalytic cleavage site, indicated by a grey inverted triangle. Used digestions were DA35 - 48h, DA55 - 48h and DA55 - 1h (Figure 1). n = 3

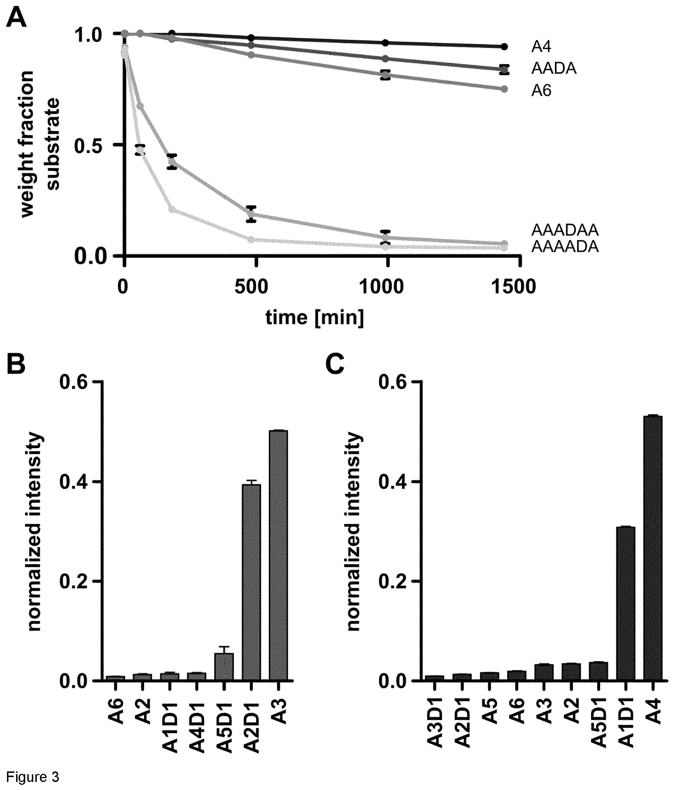

Figure 3 shows the results of different degradation experiments using the chitinosanase. A: 0.84 mg/ml of six different chitin or chitosan tetramers or hexamers (A4, AADA, A6, AAADAA and AAAADA) were digested with 0.1 mg/ml of the chitinosanase domain for 24 h. n = 3. B: Analysis of the cleavage products of the hexamer AAADAA by SEC-RI-MS. The results show that predominantly the expected fragments A3 and A2D1 are generated. C: Analysis of the cleavage products of the hexamer AAAADA by SEC-RI-MS. The results show that predominantly the expected fragments A4 and A1D1 are generated.

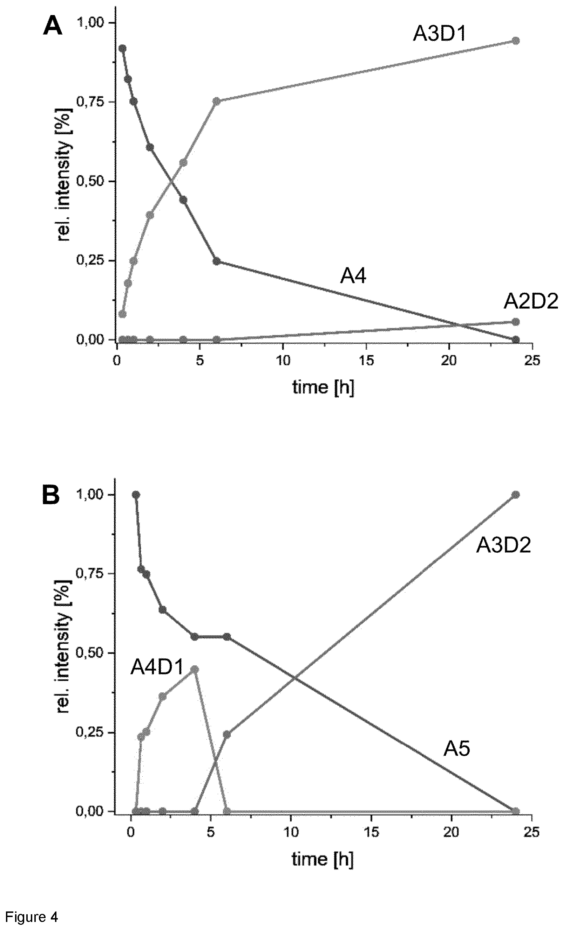

Figure 4 shows relative intensities of chitin oligomers during incubation with the chitin deacetylase domain, A) tetramer and B) pentamer, which were used as substrates in the deacetylation activity assay and resulting partially acetylated oligomers over time.
Figure 3 shows the chitin deacetylation pattern after incubation of 0.1 mg/mL of the isolated chitin deacetylase domain with 5 mM A4. Blue background represents acetylated units, blue/white background represents deacetylated units. The black cross in the respective right box represents the reducing end. The numbers on the left of the image indicate the relative abundance of the products. The numbers at the patterns show how often which pattern was found.
Figure 4 shows the chitin deacetylation pattern after incubation of 0.1 mg/mL of the isolated chitin deacetylase domain with 5 mM A5. Blue background represents acetylated units, blue/white background represents deacetylated units. The black cross in the respective right box represents the reducing end. The numbers on the left of the image indicate the relative abundance of the products. The numbers at the patterns show how often which pattern was found.

### Detailed Description

Unless otherwise defined, all terms of art, notations and other scientific terminologies used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. The techniques and procedures described or referenced herein are generally well understood and commonly employed using conventional methodology by those skilled in the art. As appropriate, procedures involving the use of commercially available kits and reagents are generally carried out in accordance with manufacturer defined protocols and/or parameters unless otherwise noted.

The term "isolated" as used herein in relation to a molecule means that said molecule has been at least partially separated from other molecules it naturally associates with or other cellular components. "Isolated" may mean that the molecule has been purified to separate it from other molecules and components, such as other proteins and nucleic acids and cellular debris.

The term "nucleic acid" as used herein includes all natural forms of nucleic acids, such as DNA and RNA. Preferably, the nucleic acid molecules of the invention are DNA.

The term "peptide" is used throughout the specification to designate a polymer of amino acid residues connected to each other by peptide bonds. A peptide according to the present invention may have 2-100 amino acid residues. The terms "protein" and "polypeptide" are used interchangeably throughout the specification to designate a polymer of amino acid residues connected to each other by peptide bonds. A protein or polypeptide according to the present invention has preferably 100 or more amino acid residues.

The term "an N-terminal fragment" relates to a peptide or protein sequence which is in comparison to a reference peptide or protein sequence C-terminally truncated, such that a contiguous amino acid polymer starting from the N-terminus of the peptide or protein remains. In some embodiments, such fragments may have a minimum length as defined herein for the polypeptides of the invention.

The term "a C-terminal fragment" relates to a peptide or protein sequence which is in comparison to a reference peptide or protein sequence N-terminally truncated, such that a contiguous amino acid polymer starting from the C-terminus of the peptide or protein remains. In some embodiments, such fragments may have a minimum length as defined herein for the polypeptides of the invention.

The term "fusion protein" as used herein concerns two or more peptides or polypeptides which are Nor C-terminally connected to each other, typically by peptide bonds, including via an amino acid/peptide linker sequence. Such fusion proteins may be encoded by two or more nucleic acid sequences which are operably fused to each other. Such fusion proteins may be artificial in that the two amino acid sequences are combined in a way that is different from any naturally occurring polypeptide.

Generally, the skilled person understands that for putting the present invention into practice any nucleotide sequence described herein may comprise an additional start and/or stop codon or that a start and/or stop codon included in any of the sequences described herein may be deleted, depending on the nucleic acid construct used. The skilled person will base this decision, e.g., on whether a nucleic acid sequence comprised in the nucleic acid molecule of the present invention is to be translated and/or is to be translated as a fusion protein. In various embodiments, the polypeptides of the invention additionally comprise the amino acid M on the N-terminus of the polypeptide.

The term "polymer" as used in the present invention is used to define molecules having more than 30 monomer units. It differs from the term "oligomer", which, in the present invention, defines molecules having 30 monomer units or less, typically 25 or less. The term "chitosan oligomers", as used herein, thus refers to molecules that comprise no more than 30 monomeric units of D-glucosamine and N-acetyl-D-glucosamine. In various embodiments, such oligomers comprise 25 monomeric units or less, 20 units or less, 18 units or less, 16 units or less or 2 to 15 units. The lower limit is the dimer, i.e. a molecule that comprises two monomeric units. Such dimers are considered oligomers in the sense of the present invention. However, in various embodiments, the oligomers of the invention comprise at least 3 units, or 4 or more units. Similarly, the term "chitosan polymer", as used herein, thus refers to molecules that comprise more than 30 monomeric units of D-glucosamine and N-acetyl-D-glucosamine, typically more than 50, more than 100 or more than 150 units. The number of units can, in various embodiments, be as high as 5000, but is typically 3000 or less, 2000 or less, 1000 or less or in the range of 200-1000, 300-1000 or 500-1000.

The term "monomer" is used in the present invention with the meaning of a constitutional unit of a polymer and interchangeably with "subunit", "monomer residue", "residue", "repeat unit" of a polymer.

The term "structural unit" as used in the present invention means a sequence of specific monomers in the polymer.

The term "chitin synthase" as used in the present invention does not limit the function of the chitin synthase enzyme to synthesizing chitin polymers but rather is intended to mean that said enzyme can synthesize chitin polymers from N-Acetyl-D-glucosamine units. The term "chitin synthase" therefore refers here to a common name given to the enzyme, discovered firstly as having chitin synthase properties.

The term "deacetylase" as used in the present invention in the term "chitin deacetylase enzyme" does not limit the function of the chitin enzyme to deacetylation but rather is intended to mean that said enzyme is capable of deacetylating a given acetylated poly- or oligosaccharide, such as chitin. It is understood that depending on the conditions, such as the concentration of acetate, the same enzyme may also have acetylating properties. This also applies to "acetylases". Accordingly, the term "chitosan acetylase" describes enzymes known for their acetylating activity on chitosan but may also have deacetylation activity.

The term "chitinase" as used in the present invention does not limit the function of the chitinase enzyme to cleaving of chitin polymers. The term "chitinase" rather refers here to an enzyme that was first discovered to have chitinase properties, for example in nature. A chitinase enzyme may thus also show chitosanase or chitinosanase properties. "Chitinosanase", as used herein, refers to an enzyme that is capable of cleaving both chitin and chitosan, typically depending on a certain motif that can be present in both types of polymers, with the motif containing at least one GlcNAc and at least one GlcN unit.

The term "chitosan polymer", as used in the present invention, defines a polymer of D-glucosamine units, wherein some D-glucosamine units may be acetylated. With other words, the term "chitosan polymer" will be used in the present invention without being limited to the fully deacetylated poly-D-glucosamine. The degree of acetylation (DA) of the chitosan polymer typically is less than 50% (otherwise, it would be termed "chitin polymer"), preferably 45 % and less, 40 % and less, 35 % and less, 30 % and less, more preferably 28% and less, 25% and less or 22% and less. The lower limit of the degree of acetylation may be 5% or more, 8% or more, 10% or more, or even 15%.

In contrast, the term "poly-D-glucosamine", as used herein, refers to the fully deacetylated chitosan that does not comprise any acetylated D-glucosamine units.

The term "chitin", as used herein, refers to a polymer comprising *N*-acetyl-D-glucosamine and D-glucosamine units, with the degree of acetylation being at least 50%, typically at least 70% or at least 80%. The term "poly-*N*-acetyl-D-glucosamine" is then used to designate a chitin that comprises 100% acetylated monomer units.

The term "GlcN" or "D" as used in the present invention stands for 2-amino-2-deoxy-beta-D-glucose or D-glucosamine. If reference is made herein to "glucosamine", D-glucosamine is meant if not indicated otherwise.

The term "GlcNAc" or "A" as used in the present invention stands for 2-acetamido-2-deoxy-beta-D-glucose or *N*-acetyl-D-glucosamine. If reference is made herein to "*N*-acetyl-glucosamine", *N*-Acetyl-D-glucosamine is meant if not indicated otherwise.

The term "degree of acetylation" or "DA", as used herein, is calculated as ΣA/(ΣA+ΣD)*100 to give the DA in percent (%), wherein in this formula ΣA is the number of GlcNAc units and ΣD the number of GlcN units in a given molecule. The degree of acetylation is, for example, determined by NMR, for example 1H-NMR or 13C-NMR, preferably 1H-NMR. Additionally or alternatively, the degree of acetylation can be determined by means of enzymatic hydrolysis of the chitosan polymer to monomers or small oligomers and quantitative mass spectrometry analysis. Methods to enzymatically hydrolyze a chitosan polymer are known in the art and can be chosen by those skilled in the art based on their general knowledge. In the case of chitosan oligomers, the preferred method for DA determination is mass spectrometry, but other methods such as, but not limited to NMR, Thin Layer Chromatography (TLC), or electrophoresis are also available. Still other methods include titration, dye binding assays, UV spectroscopy, and (FT)IR spectroscopy.

The term "degree of polymerization" or "DP", as used herein, refers to the number of monomeric units in a polymer or oligomer molecule. For polymers, it may also be given as the number average degree of polymerization which is given as DPₙ = Mₙ/M₀ with Mₙ being the number average molecular weight of the polymer and M₀ being the molecular weight of a monomeric unit. The DP of chitosan polymers may be determined by Size Exclusion Chromatography (SEC), preferably SEC-RI-MALLS. The DP of chitosan oligomers may be determined by TLC, NMR, or, preferably, mass spectrometry.

The molecular weight of a polymer is, if not indicated otherwise, typically the number average molecular weight (Mₙ). This can be determined by a variety of methods known to those skilled in the art, such as, without limitation, GPC. In various embodiments, the molecular weight may also be determined using the method described in Niehues et al. ("Chitosan analysis by enzymatic / mass spectrometric fingerprinting and in silico predictive modeling", Analytical Chemistry (2017) 89(22):12602-12608, by reference to the method described in C. Schatz, C. Viton, T. Delair, C. Pichot, and A. Domard, "Typical physicochemical behaviors of chitosan in aqueous solution", Biomacromolecules, (2003) 4: 641-648).

The term "about", as used in the context of the present invention, is intended to mean the referenced numerical value ± 10 %, preferably ± 5 %.

Determination of the sequence identity of nucleic acid or amino acid sequences can be done by a sequence alignment based on well-established and commonly used BLAST algorithms (See, e.g. Altschul, S.F., Gish, W., Miller, W., Myers, E.W. & Lipman, D.J. (1990) "Basic local alignment search tool." J. Mol. Biol. 215:403-410, and Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402). Such an alignment is based on aligning similar nucleotide or amino acid sequences stretches with each other. Another algorithm known in the art for said purpose is the FASTA algorithm. Alignments, in particular multiple sequence comparisons, are typically done by using computer programs. Commonly used are the Clustal series (See, e.g., Chenna et al. (2003): Multiple sequence alignment with the Clustal series of programs. Nucleic Acid Research 31, 3497-3500), T-Coffee (See, e.g., Notredame et al. (2000): T-Coffee: A novel method for multiple sequence alignments. J. Mol. Biol. 302, 205-217) or programs based on these known programs or algorithms.

Also possible are sequence alignments using the computer program Plasmid NTI^{®} Suite 10.3 (Invitrogen Corporation, 1600 Faraday Avenue, Carlsbad, CA, USA) with the set standard parameters, with the AlignX module for sequence comparisons being based on the ClustalW. If not indicated otherwise, the sequence identity is determined using the BLAST algorithm.

Such a comparison allows determination of the similarity of the compared sequences. Said similarity is typically expressed in percent identify, i.e. the portion of identical nucleotides/amino acids at the same or corresponding (in an alignment) sequence positions relative to the total number of the aligned nucleotides/amino acids. For example, if in an alignment 90 amino acids of a 100 aa long query sequence are identical to the amino acids in corresponding positions of a template sequence, the sequence identity of the query sequence is 90%. The broader term "homology" additionally considers conserved amino acid substitutions, i.e. amino acids that are similar in regard to their chemical properties, since those typically have similar chemical properties in a protein. Accordingly, such homology can be expressed in percent homology. If not indicated otherwise, sequence identity and sequence homology relate to the entire length of the aligned sequence.

The amino acids are typically referred to herein in the one-letter code. The indication "D14" thus refers to the aspartic acid residue in position 14.

The amino acids in the positions that correspond to positions D111 and E113 (of SEQ ID NO:1), D6, D7, H57, H61, H164, R95 Y98, D129, D132 and W133 (of SEQ ID NO: 2) and D88, D90, D180, R219 and W220 (SEQ ID NO:3) are preferably invariable, as these are considered to be involved in enzymatic activity. These amino acids are generally conserved in the polypeptides of the invention that comprise any one or more of SEQ ID Nos. 1-3, including SEQ ID NO: 4. "Invariable", as used herein, generally means that the respective amino acid at the indicated position may not be changed, i.e. substituted or deleted.

The present invention is based on the finding of the inventors that a novel three-domain transmembrane enzyme having the amino acid sequence of SEQ ID NO:4, comprises all three of a transmembrane chitin synthase domain (SEQ ID NO: 3), a chitin deacetylase domain (SEQ ID NO: 2) and a chitinase domain (SEQ ID NO: 1). This arrangement has been newly found, since these type of enzymes have not been found together on a single polypeptide chain. It was found that not only do the individual domains have good and in the case of SEQ ID NO:1 previously unknown activity and specificity, but they can be expressed together and act together, thus facilitating the previously impossible recombinant production of chitosan. This becomes possible by the specific arrangement of a chitin synthase and a chitin deacetylase on the same polypeptide chain, which allows that the nascent or freshly produced chitin is deacetylated before it forms fibrils, which are typically no longer susceptible to the enzymatic deacetylation activity of a polypeptide separately expressed, i.e. expressed as a separate polypeptide.

Thus, in a first aspect, the present invention relates to an isolated polypeptide comprising an amino acid sequence having at least 80%, such as at least 81%, 82%, 83%, 84%, 85%, 86%, 87%, 88% or 89%, 90%, 91%, 92%, 93%, 94%, 95%, 96%, 97%, 98% or 99%, preferably 100% sequence identity over its entire length with the amino acid sequence set forth in SEQ ID NO:1. Said isolated polypeptide is preferably a functional enzyme in that it has chitinase activity, preferably chitinosanase activity.

"Chitinosanase activity", as used in this context, refers to the capability of the enzyme to cleave both chitin and chitosan molecules as long as they have the necessary motif, as described below.

The functionality of the isolated polypeptide may be such that it has chitinase activity, preferably chitinosanase activity, which under identical conditions, is at least 60%, at least 70%, or at least 80%, for example at least 81%, 82%, 83%, 84%, 85%, 86%, 87%, 88% or 89%, 90%, 91%, 92%, 93%, 94%, 95%, 96%, 97%, 98% or 99%, preferably 100% or more relative to the chitinosanase activity of a polypeptide consisting of the full-length sequence of SEQ ID NO:4.

The polypeptide having chitinosanase activity typically cleaves a partially deacetylated chitin polymer or a chitosan polymer comprising the motif:

[X]ₙAA | D[X]ₙ,

wherein X is Glucosamine (GlcN) or *N*-Acetyl-Glucosamine (GlcNAc), A is *N*-Acetyl-Glucosamine (GlcNAc), D is Glucosamine (GIcN), each n is independently 0 or an integer of from 1 to 5000, preferably 1 to 1000, and | indicates the cleavage site/position. The enzyme thus recognizes a motif of at least two acetylated glucosamine units followed by at least one deacetylated glucosamine unit and cleaves between the acetylated and the deacetylated unit.

All chitin and chitosan polymers are shown herein from non-reducing to reducing end (left to right).

The chitosan polymers or oligomers obtained by using the isolated polypeptide having chitinosanase activity therefore have the following structure:

D(X)ₙAA

wherein D is Glucosamine (GIcN), A is *N*-Acetyl-Glucosamine (GlcNAc), X is Glucosamine (GlcN) or *N-*Acetyl-Glucosamine (GlcNAc) and n is independently 0 or an integer of from 1 to 100, preferably 0 to 50, 0 to 30, 0 to 20, 0 to 15, or 0 to 10, and wherein (X)ₙ does optionally not comprise the unit AAD. If the product does comprise the unit AAD, it may still serve as a substrate for the chitinosanase for another round of cleavage. However, in some embodiments, it may be desirable to tune the cleavage conditions such that the cleavage reaction is incomplete, as this allows to fine tune the molecular weight of the chitosan polymers/oligomers obtained. The thus obtained mixtures of chitosan oligomers/polymers are characterized by their specific structure with a deacetylated glucosamine on the non-reducing end and two acetylated glucosamines on the reducing end.

The thus generated chitosans also form part of the present invention. Due to the relatively high specificity of the enzyme and the motif recognized and cleaved, the chitosan mixture thus obtained is structurally highly defined and uniform.

In various embodiments, said isolated polypeptide does not comprise the full-length amino acid sequence of SEQ ID NO:4, i.e. differs from said full length polypeptide in that it lacks certain parts of the full length polypeptide of SEQ ID NO:4. Alternatively or additionally, it comprises parts that do not naturally occur in said polypeptide of SEQ ID NO:4, i.e. either by fusion of parts non-naturally associated with the full length polypeptide or by mutation.

The amino acid sequence of said polypeptide of the first aspect of the present invention is typically up to 1000 amino acids in length, preferably up to 900, up to 800, up to 700, up to 600, up to 500, up to 400 or up to 300 and/or optionally at least 250 amino acids in length, preferably at least 270 and even more preferably at least 290 amino acids in length. The range may thus be from 250 to 1000 amino acids, 250 to 900, 250 to 800, 250 to 700, 250 to 600, 270 to 500, 270 to 400 or 290 to 350 or 290 to 300 amino acids.

The amino acids (corresponding to) D111 and E113, using the positional numbering of SEQ ID NO:1, are typically invariable, as they are considered essential for enzymatic activity. Accordingly, in the polypeptides of the third aspect, one or both of these amino acids may be invariable.

The polypeptide according to the first aspect may have additional amino acid sequences that flank the amino acid sequence that has at least 80% sequence identity to SEQ ID NO:1. These are referred to herein as "flanking sequences". The flanking sequences may be present on the N- and/or the C-terminus and may be of varying length. However, in various embodiments, the length limits described above are preferably also met for a polypeptide including such flanking sequences.

The N-terminal flanking sequence may have the amino acid sequence of SEQ ID NO:10 or may be any C-terminal fragment thereof. In various embodiments, said fragment is up to 90, up to 80, up to 70, up to 60, up to 50, up to 40, up to 30, or up to 20 amino acids in length. In some embodiments, it is a 52 amino acid long C-terminal fragment of SEQ ID NO:10, as used in the construct utilized in the examples (SEQ ID NO:12). "C-terminal fragment", as used herein, relates to a given sequence that lacks amino acids from its N-terminus relative to a starting or reference sequence. Accordingly, "N-terminal fragment", as used herein, relates to a given sequence that lacks amino acids from its C-terminus relative to a starting or reference sequence.

The C-terminal flanking sequence may have the amino acid sequence of SEQ ID NO:8 or may be any N-terminal fragment thereof. In various embodiments, said fragment is up to 75, up to 70, up to 60, up to 50, up to 40, up to 30, or up to 20 amino acids in length. In some embodiments, it is a 20 amino acid long N-terminal fragment of SEQ ID NO:8, as used in the construct utilized in the examples (SEQ ID NO:12). If the C-terminal flanking sequence comprises the full amino acid sequence of SEQ ID NO:8, it can further comprise part or the full length sequence of SEQ ID NO:2 attached to the C-terminus of SEQ ID NO:8.

In a second aspect, the invention is directed to the part of the newly identified polypeptide that has chitin deacetylase activity. In the full length protein, this is the middle domain. The present invention therefore relates to an isolated polypeptide comprising an amino acid sequence having at least 80%, such as at least 81%, 82%, 83%, 84%, 85%, 86%, 87%, 88% or 89%, 90%, 91%, 92%, 93%, 94%, 95%, 96%, 97%, 98% or 99%, preferably 100% sequence identity over its entire length with the amino acid sequence set forth in SEQ ID NO:2. Said polypeptide typically possesses chitin deacetylase activity. It is preferred that such a polypeptide has chitin deacetylase activity, which is, under identical conditions, at least 60%, at least 70%, at least 80%, at least 81%, 82%, 83%, 84%, 85%, 86%, 87%, 88% or 89%, 90%, 91%, 92%, 93%, 94%, 95%, 96%, 97%, 98% or 99 %, preferably 100% or more relative to the deacetylase activity of a polypeptide consisting of the full-length sequence of SEQ ID NO:4.

In various embodiments, said isolated polypeptide does not comprise the full-length amino acid sequence of SEQ ID NO:4, i.e. differs from said full length polypeptide in that it lacks certain parts of the full length polypeptide of SEQ ID NO:4. Alternatively or additionally, it comprises parts that do not naturally occur in said polypeptide of SEQ ID NO:4, i.e. either by fusion of parts non-naturally associated with the full length polypeptide or by mutation.

The polypeptide of the second aspect of the present invention is typically up to 1000 amino acids in length, preferably up to 900, up to 800, up to 700, up to 600, up to 500, up to 400 up, to 300, up to 250 or up to 200 and/or at least 180 amino acids in length, preferably at least 190 and even more preferably at least 195 amino acids in length. The range may thus be from 180 to 1000 amino acids, 180 to 900, 180 to 800, 180 to 700, 180 to 600, 190 to 500, 190 to 400 or 195 to 300 or 195 to 250 or 195 to 220 or 195 to 200 amino acids.

The amino acids (corresponding to) D6, D7, H57, H61 and H164, using the positional numbering of SEQ ID NO:2, are preferably invariable, as they are considered essential for enzymatic activity. In various embodiments, this also extends to the amino acids (corresponding to the following residues): R95, Y98, D129, D132 and W133, using the numbering according to SEQ ID NO:2. Accordingly, in the polypeptides of the second aspect, any one or more, or all of these amino acids may be invariable.

The polypeptide according to the second aspect may have additional amino acid sequences that flank the amino acid sequence that has at least 80% sequence identity to SEQ ID NO:2. The flanking sequences may be present on the N- and/or the C-terminus and may be of varying length. However, in various embodiments, the length limits described above are preferably also met for a polypeptide including such flanking sequences.

The N-terminal flanking sequence may have the amino acid sequence of SEQ ID NO:8 or may be any C-terminal fragment thereof. In various embodiments, said fragment is up to 75, up to 70, up to 60, up to 50, up to 40, up to 30, or up to 20 amino acids in length. In some embodiments, it is a 18 amino acid long C-terminal fragment of SEQ ID NO:8, as used in the construct utilized in the examples (SEQ ID NO:13). If the N-terminal flanking sequence comprises the full amino acid sequence of SEQ ID NO:8, it can further comprise part or the full length sequence of SEQ ID NO:1 attached to the N-terminus of SEQ ID NO:8.

The C-terminal flanking sequence may have the amino acid sequence of SEQ ID NO:9 or may be any N-terminal fragment thereof. In various embodiments, said fragment is up to 70, up to 60, up to 50, up to 40, up to 30, or up to 20 amino acids in length. In some embodiments, it is a 13 amino acid long N-terminal fragment of SEQ ID NO:9, as used in the construct utilized in the examples (SEQ ID NO:13). If the C-terminal flanking sequence comprises the full amino acid sequence of SEQ ID NO:9, it can further comprise part or the full length sequence of SEQ ID NO:3 attached to the C-terminus of SEQ ID NO:9.

In a third aspect, the present invention is directed to the part of the polypeptide found that has chitin synthase activity. This is the most N-terminal domain of the native protein. In this aspect, the present invention therefore relates to an isolated polypeptide comprising an amino acid sequence having at least 80%, such as at least 81%, 82%, 83%, 84%, 85%, 86%, 87%, 88% or 89%, 90%, 91%, 92%, 93%, 94%, 95%, 96%, 97%, 98% or 99%, preferably 100% sequence identity over its entire length with the amino acid sequence set forth in SEQ ID NO:1. SEQ ID NO:3. Said isolated polypeptide typically has enzymatic activity, in particular chitin synthase activity.

It is preferred that such a polypeptide has chitin synthase activity, which is, under identical conditions, at least 60%, at least 70%, at least 80%, at least 81%, 82%, 83%, 84%, 85%, 86%, 87%, 88% or 89%, 90%, 91%, 92%, 93%, 94%, 95%, 96%, 97%, 98% or 99%, preferably 100% or more relative to the chitin synthase activity of a polypeptide consisting of the full-length sequence of SEQ ID NO:4.

In various embodiments, said isolated polypeptide does not comprise the full-length amino acid sequence of SEQ ID NO:4, i.e. differs from said full length polypeptide in that it lacks certain parts of the full length polypeptide of SEQ ID NO:4. Alternatively or additionally, it comprises parts that do not naturally occur in said polypeptide of SEQ ID NO:4, i.e. either by fusion of parts non-naturally associated with the full length polypeptide or by mutation.

The polypeptide of the third aspect of the present invention is typically up to 1000 amino acids in length, preferably up to 900, up to 800, up to 700, up to 600, up to 500, up to 400, up to 300, or up to 250 and/or at least 200 amino acids in length, preferably at least 210 and even more preferably at least 220 amino acids in length. The range may thus be from 200 to 1000 amino acids, 200 to 900, 200 to 800, 200 to 700, 200 to 600, 210 to 500, 210 to 400 or 210 to 300 or 220 to 250 amino acids.

The amino acids at the positions corresponding to D88, D90, D180, R219 and W220, using the positional numbering according to SEQ ID NO:3, are preferably invariable, as they are considered essential for enzymatic activity. Accordingly, in the polypeptides of the third aspect, any one or more, or all of these amino acids may be invariable.

The polypeptide according to the third aspect may have additional amino acid sequences that flank the amino acid sequence that has at least 80% sequence identity to SEQ ID NO:3. The flanking sequences may be present on the N- and/or the C-terminus and may be of varying length. However, in various embodiments, the length limits described above are preferably also met for a polypeptide including such flanking sequences.

The N-terminal flanking sequence may have the amino acid sequence of SEQ ID NO:9 or may be any C-terminal fragment thereof. In various embodiments, said fragment is up to 70, up to 60, up to 50, up to 40, up to 30, or up to 20 amino acids in length. If the C-terminal flanking sequence comprises the full amino acid sequence of SEQ ID NO:9, it can further comprise part or the full length sequence of SEQ ID NO:2 attached to the N-terminus of SEQ ID NO:9.

The C-terminal flanking sequence may have the amino acid sequence of SEQ ID NO:11 or may be any N-terminal fragment thereof. In various embodiments, said fragment is up to 140, up to 120, up to 100, up to 80, up to 60, up to 50, up to 40, up to 30, or up to 20 amino acids in length.

In another aspect, the present invention relates to an isolated polypeptide which comprises any two or three of the polypeptides of the first, second and third aspect of the invention. Accordingly, it comprises amino acid sequences that have at least 80%, such as at least 81%, 82%, 83%, 84%, 85%, 86%, 87%, 88% or 89%, 90%, 91%, 92%, 93%, 94%, 95%, 96%, 97%, 98% or 99%, or 100% sequence identity over their entire length with the amino acid sequence set forth in (1) SEQ ID NO:1, (2) in SEQ ID NO:2 and/or (3) SEQ ID NO:3. As these sequences do not need to be directly adjacent to each other, the given sequence identities relate to the respective part of the polypeptide of the invention. For example, if the polypeptide comprises a sequence that has at least 80% sequence identity to SEQ ID NO:2 and another sequence that has at least 80% sequence identity to SEQ ID NO:3 and both are connected by a linking sequence that has no similarity to each of SEQ ID NO:2 and SEQ ID NO:3, it is understood that such polypeptide would still comprise an amino acid sequence that has at least 80% sequence identity to SEQ ID NO:2 and another sequence that has at least 80% sequence identity to SEQ ID NO:3.

In various embodiments, the isolated polypeptide comprises (1) and (2) but not (3), (2) and (3) but not (1), (1) and (3) but not (2) or (1), (2) and (3). This means that the isolated polypeptide may comprise the chitinosanase domain together with the chitin deacetylase domain or the chitin synthase domain or both or the polypeptide may comprise the chitin synthase domain and the chitin deacetylase domain. It is understood that this also covers embodiments, where the polypeptides cover only a part of any of the other domains. However, all polypeptide of the invention preferably comprise at least one of the functional domains described herein.

The individual domains may be separated by a linker amino acid sequence. Said linker amino acid sequence may, in various embodiments, be up to 80 amino acids in length and is connected to the C- and N-terminus of the two respective domains that are linked to it by normal peptide bonds.

The respective linker sequences may be derived from the full length sequence of SEQ ID NO:4, i.e. the linker sequence between the domain of SEQ ID NO:1 or a variant thereof and the domain of SEQ ID NO:2 or a variant thereof may for example have the amino acid sequence of SEQ ID NO:8 or be a fragment thereof, wherein said fragment of SEQ ID NO:8 may be an N- and/or C-terminally truncated version of SEQ ID NO:8. However, it is also possible to replace this native linker sequence by an artificial linker sequence, for example a linker sequence that comprises a protease recognition site.

Similarly, the linker sequence between the domain of SEQ ID NO:2 or a variant thereof and the domain of SEQ ID NO:3 or a variant thereof may for example have the amino acid sequence of SEQ ID NO:9 or be a fragment thereof, wherein said fragment of SEQ ID NO:9 may be an N- and/or C-terminally truncated version of SEQ ID NO:9. However, it is also possible to replace this native linker sequence by an artificial linker sequence, for example a linker sequence that comprises a protease recognition site.

The term "variant", as used above, relative to those polypeptides that haver at least 80% but not 100% sequence identity to the respective reference sequence.

In this aspect of the invention, the polypeptide may again comprise flanking sequences as described herein above. The respective flanking sequences, if arranged between two domains, are also termed linker sequences and are defined as described above. The flanking sequence of SEQ ID NO:10 which is located N-terminal to SEQ ID NO:1 in the native sequence (as set forth in SEQ ID NO:4) and the flanking sequence of SEQ ID NO:11 which is located C-terminal to SEQ ID NO:3 in the native sequence are true flanking sequences as they are typically not located between two domains and are thus not linker sequences. In contrast, the sequences of SEQ ID NO:8 and SEQ ID NO:9, which are located between SEQ ID NO:1 and 2 and SEQ ID NO:2 and 3, respectively, are either flanking sequences or linker sequences depending on whether the polypeptide of the invention comprises one or more domains selected from SEQ ID NOs. 1-3.

In various embodiments, the isolated polypeptide comprises any one of the amino acid sequences of SEQ ID Nos. 5-7 or a variant thereof that has at least 80%, at least 85%, or at least 90%, such as at least 81%, 82%, 83%, 84%, 85%, 86%, 87%, 88% or 89%, 90%, 91%, 92%, 93%, 94%, 95%, 96%, 97%, 98% or 99%, or 100% sequence identity over its entire length with any one of SEQ ID Nos. 5-7. SEQ ID NO:5 corresponds to SEQ ID NO:1 - SEQ ID NO:8 - SEQ ID NO:2, in this particular order from N- to C-terminus. SEQ ID NO:6 corresponds to SEQ ID NO:2 - SEQ ID NO:9 - SEQ ID NO:3 and SEQ ID NO:7 corresponds to SEQ ID NO:1 - SEQ ID NO:8 - SEQ ID NO:2 - SEQ ID NO:9 - SEQ ID NO:3.

In some embodiments, the polypeptides may be derivatized or conjugated to another chemical moiety. In various embodiments, this may also be an amino acid tag, for example to facilitate purification, such as by affinity chromatography. Such peptide tags include the known 6xHis tag or longer tags such as a GST tag or a Strep tag. Alternative chemical moieties include, without limitation, biotin.

The invention further relates to nucleic acids, in particular isolated nucleic acid molecules, encoding any of the polypeptides of the present invention, as herein described above. Such nucleic acid molecules may comprise or consist of the nucleotide sequence set forth in any one of SEQ ID Nos. 15 to 19, wherein SEQ ID Nos. 15 and 16 encode for polypeptides comprising or consisting of SEQ ID NO:1, SEQ ID Nos. 17 and 18 encode for polypeptides comprising or consisting of SEQ ID NO:2, and SEQ ID NO:19 encodes for a polypeptide consisting of SEQ ID NO:3.

If any polypeptide comprises in addition to the amino acid sequence specified herein optional further amino acid sequences, all of these amino acid sequences are typically linked by peptide bonds and expressed as a single fusion protein. To facilitate said expression, the nucleic acid molecule comprises nucleotide sequences encoding all amino acid sequences, with said nucleotide sequences being operably linked to allow expression of the single fusion protein comprising all afore-mentioned amino acid sequences.

The term "operably linked" in the context of nucleic acid sequences means that a first nucleic acid sequence is linked to a second nucleic acid sequence such that the first nucleic acid sequence is placed in a functional relationship with the second nucleic acid sequence. For instance, a promoter sequence is operably linked to a coding sequence of a heterologous gene if the promoter can initiate the transcription of the coding sequence. In a further context, a sequence encoding the polypeptide of the present invention is linked such to another amino acid sequence, that if the two sequences are translated a single peptide/protein chain is obtained.

In certain embodiments, the above defined nucleic acid molecules may be comprised in a vector, for example a cloning or expression vector. Generally, the nucleic acid molecules of the invention can also be part of a vector or any other kind of cloning vehicle, including, but not limited to a plasmid, a phagemid, a phage, a baculovirus, a cosmid, or an artificial chromosome. Generally, a nucleic acid molecule disclosed in this application may be "operably linked" to a regulatory sequence (or regulatory sequences) to allow expression of this nucleic acid molecule. Examples of regulatory systems are those that cause expression of the gene to be turned on or off in response to a chemical or physical stimulus, including the presence of a regulatory compound. Non-limiting examples of regulatory systems in prokaryotic systems include the lac, tac, and trp operator systems.

Such cloning vehicles can include, besides the regulatory sequences described above and a nucleic acid sequence of the present invention, replication and control sequences derived from a species compatible with the host cell that is used for expression as well as selection markers conferring a selectable phenotype on transformed or transfected cells. Large numbers of suitable cloning vectors are known in the art and are commercially available.

In certain embodiments the nucleic acid molecules disclosed herein are comprised in a cloning vector.

Therefore, in a further aspect, the present invention also relates to a vector comprising a nucleic acid molecule according to the present invention, wherein preferably the vector is a plasmid.

In some embodiments the nucleic acid molecules disclosed herein are comprised in an expression vector. The vectors may comprise regulatory elements for replication and selection markers. In certain embodiments, the selection marker may be selected from the group consisting of genes conferring ampicillin, kanamycin, chloramphenicol, tetracycline, blasticidin, spectinomycin, gentamicin, hygromycin, and zeocin resistance. In various other embodiments, the selection may be carried out using antibiotic-free systems, for example by using toxin/antitoxin systems, cer sequence, triclosan, auxotrophies or the like. Suitable methods are known to those skilled in the art.

The above-described nucleic acid molecule of the present invention, comprising a nucleic acid sequence encoding for the polypeptide of the invention, if integrated in a vector, must be integrated such that the polypeptide can be expressed. Therefore, a vector of the present invention comprises sequence elements which contain information regarding to transcriptional and/or translational regulation, and such sequences are "operably linked" to the nucleotide sequence encoding the polypeptide. An operable linkage in this context is a linkage in which the regulatory sequence elements and the sequence to be expressed are connected in a way that enables gene expression. The precise nature of the regulatory regions necessary for gene expression may vary among species, but in general these regions comprise a promoter which, in prokaryotes, contains both the promoter per se, i.e., DNA elements directing the initiation of transcription, as well as DNA elements which, when transcribed into RNA, will signal the initiation of translation. Such promoter regions normally include 5' non-coding sequences involved in initiation of transcription and translation, such as the -35/-10 boxes and the Shine-Dalgarno element in prokaryotes or the TATA box, CAAT sequences, and 5'- capping elements in eukaryotes. These regions can also include enhancer or repressor elements as well as translated signal and leader sequences for targeting the native polypeptide to a specific compartment of a host cell.

In addition, the 3' non-coding sequences may contain regulatory elements involved in transcriptional termination, polyadenylation or the like. If, however, these termination sequences are not satisfactorily functional in a particular host cell, then they may be substituted with signals functional in that cell.

In various embodiments, a vector comprising a nucleic acid molecule of the invention can therefore comprise a regulatory sequence, preferably a promoter sequence. In certain embodiments, the promoter is identical or homologous to promoter sequences of the host genome. In such cases endogenous polymerases may be capable to transcribe the nucleic acid molecule sequence comprised in the vector. In various embodiments, the promoter is selected from the group of weak, intermediate and strong promoters, preferably from weak to intermediate promoters.

In another preferred embodiment, a vector comprising a nucleic acid molecule of the present invention comprises a promoter sequence and a transcriptional termination sequence. Suitable promoters for prokaryotic expression are known in the art and include, for example and without limitation, the araBAD promoter, the tet-promoter, the lacUV5 promoter, the CMV promo tor, the EF1 alpha promotor, the AOX1 promotor, the tac promotor, the T7promoter, or the lac promotor.

Furthermore, a nucleic acid molecule of the invention can comprise transcriptional regulatory elements, e.g., repressor elements, which allow regulated transcription and translation of coding sequences comprised in the nucleic acid molecule. Repressor element may be selected from the group consisting of the Lac-, AraC-, or MalR-repressor.

The vector may be effective for prokaryotic or eukaryotic protein expression, preferably prokaryotic expression. Suitable vectors are known to those skilled in the art.

The vectors of the present invention may be chosen from the group consisting of high, medium and low copy vectors.

The above-described vectors of the present invention may be used for the transformation or transfection of a host cell in order to achieve expression of a peptide or protein which is encoded by an above-described nucleic acid molecule and comprised in the vector DNA.

Thus, in a further aspect, the present invention also relates to a host cell comprising a vector or nucleic acid molecule as disclosed herein.

Also contemplated herein are host cells, which comprise a nucleic acid molecule as described herein integrated into their genomes. The skilled person is aware of suitable methods for achieving the nucleic acid molecule integration. For example, the molecule may be delivered into the host cells by means of liposome transfer or viral infection, electroporation, microinjection or heat shock treatment and afterwards the nucleic acid molecule may be integrated into the host genome by means of homologous recombination. In certain embodiments, the nucleic acid molecule is integrated at a site in the host genome, which mediates transcription of the peptide or protein of the invention encoded by the nucleic acid molecule. In various embodiments, the nucleic acid molecule further comprises elements which mediate transcription of the nucleic acid molecule once the molecule is integrated into the host genome and/or which serve as selection markers.

In certain embodiments, the nucleic acid molecule of the present invention is transcribed by a polymerase natively encoded in the host genome. In various embodiments, the nucleic acid molecule is transcribed by an RNA-polymerase which is non-native to the host genome. In such embodiments, the nucleic acid molecule of the present invention may further comprise a sequence encoding for a polymerase and/or the host genome may be engineered or the host cell may be infected to comprise a nucleic acid sequence encoding for an exogenous polymerase. The host cell may be specifically chosen as a host cell capable of expressing the gene. In addition or otherwise, in order to produce the isolated polypeptide of the invention, the nucleic acid coding for it can be genetically engineered for expression in a suitable system. Transformation can be performed using standard techniques (Sambrook, J. et al. (2001), Molecular Cloning: A Laboratory Manual, 3rd Ed., Cold Spring Harbor Laboratory Press, Cold Spring Harbor, NY).

Prokaryotic or eukaryotic host organisms comprising such a vector for recombinant expression of the polypeptide as described herein form also part of the present invention. Suitable host cells can be prokaryotic cells. In certain embodiments the host cells are selected from the group consisting of gram positive and gram-negative bacteria. In some embodiments, the host cell is a gram-negative bacterium, such as Escherichia coli (E. coli). In certain embodiments, the host cell is E. coli. In further embodiments, the host cell is selected from the group consisting of E. coli, Pseudomonas, Serratia marcescens, Salmonella, Shigella (and other enterobacteriaceae), Neisseria, Hemophilus, Klebsiella, Proteus, Enterobacter, Helicobacter, Acinetobacter, Moraxella, Stenotrophomonas, Bdellovibrio, Vibrio, Legionella, acetic acid bacteria, Bacilli, Paenibacilli, Corynebacterium, Clostridium, Listeria, Streptococcus, Staphylococcus, and Archaea cells. Suitable eukaryotic host cells are among others CHO cells, insect cells, fungi, yeast cells, e.g., Saccharomyces cerevisiae, S. pombe, Pichia pastoris, and the like.

In some embodiments, the host cell is selected from the group consisting of E. coli, in particular E. coli BL21 (DE3), E. coli BL21, E. coli K12, E. coli BLR, E. coli BL21 AI, E. coli BL21 pLysS, E. coli XL1, E. coli DH5a, E. coli DH1, E. coli DM1, E. coli HB101, E. coli JmIOI-110, E. coli Rosetta(DE3)pLysS, E. coli SURE, E. coli TOP10, E. coli XLI-Blue, E. coli XL2-Blue, and E. coli XLIO-Blue.

The transformed host cell described herein is cultured under conditions suitable for expression of the nucleotide sequence encoding the polypeptides of the invention. In certain embodiments, the cell is cultured under conditions suitable for expression of the nucleotide sequence encoding a polypeptide of the invention and, optionally, its integration into the host cell's membrane.

For producing the polypeptide of the present invention, a vector of the invention can be introduced into a suitable prokaryotic or eukaryotic host organism by means of recombinant DNA technology (as already outlined above). For this purpose, the host cell is first transformed with a vector comprising a nucleic acid molecule according to the present invention using established standard methods (Sambrook, J. et al. (2001), supra). The host cell is then cultured under conditions, which allow expression of the heterologous DNA and thus the synthesis of the corresponding polypeptide. Subsequently, the polypeptide may be recovered either from the cell or from the cultivation medium. Alternatively and preferably, the recombinant host cell expressing the polypeptides of the invention is then cultivated under conditions that allow the synthesis of chitin or chitosan, as desired.

For expression of the polypeptides of the present invention several suitable protocols are known to the skilled person. The method for expression of a recombinant polypeptide of the present invention may be achieved by the following method comprising: (a) introducing a nucleic acid molecule or vector of the invention into a host cell, wherein the nucleic acid molecule or vector encodes the polypeptide of the present invention; and (b) cultivating the host cell in a culture medium under conditions that allow expression of the polypeptide of the present invention.

Step (a) may be carried out by using suitable transformation and transfection techniques known to those skilled in the art. These techniques are usually selected based on the type of host cell into which the nucleic acid is to be introduced. In some embodiments, the transformation may be achieved using electroporation or heat shock treatment of the host cell.

Step (b) may include a cultivation step that allows growth of the host cells. Alternatively, such step allowing growth of the host cells and a step that allows expression of the polypeptide may be performed separately in that the cells are first cultivated such that they grow to a desired density and then they are cultivated under conditions that allow expression of the polypeptide. The expression step can however still allow growth of the cells.

The method may further include a step of recovering the expressed polypeptide. The polypeptide may be recovered from the growth medium, if it is secreted, or from the cells or both. The recovery of the polypeptide may include various purification steps.

Generally, any known culture medium suitable for growth of the selected host may be employed in this method.

In various embodiments, the method may also encompass the purification the polypeptide, wherein the polypeptide is purified using a method selected from affinity chromatography, ion exchange chromatography, reverse phase chromatography, size exclusion chromatography, and combinations thereof.

In a further aspect, the present invention relates to the use of a vector or nucleic acid molecule as disclosed herein for the expression of a polypeptide according to the present invention. In some embodiments, the vector is used for the expression.

In another embodiment, the present invention relates to the use of a polypeptide, as herein described and defined, for the biotechnological recombinant production of chitosans comprising cultivating a host cell as described above, wherein the host cell comprises a nucleic acid molecule encoding a polypeptide as defined herein above, for example a polypeptide comprising an amino acid sequence that has at least 80%, such as at least 81%, 82%, 83%, 84%, 85%, 86%, 87%, 88% or 89%, 90%, 91%, 92%, 93%, 94%, 95%, 96%, 97%, 98% or 99%, or 100% sequence identity over its entire length with the amino acid sequence set forth in any one or both SEQ ID Nos.1 and/or 2, under conditions that allow the functional expression of the encoded polypeptide. Said polypeptide may further comprise the functional sequence of SEQ ID NO:3 or a variant thereof. In this case, the synthesis of the chitosans occurs such that first chitin is produced by the chitin synthase of SEQ ID NO:3 or a variant thereof, the chitin is then deacetylated by the chitin deacetylase of SEQ ID NO:2 or fragment thereof, and the chitosans produced are then cleaved by the chitinosanase of SEQ ID NO:1 or fragment thereof. If no fragmentation of the chitosans is desired, the cell may only comprise the polypeptide of SEQ ID NO:2 and, optionally, SEQ ID NO:3, in which case the chitosan produced by the chitin deacetylase is not cleaved in shorter fragments.

In various embodiments, said method comprises the secretion of chitosan into the extracellular space and optionally the step of isolating the chitosan from culture medium. This may occur if the polypeptide is inserted into the host cell's membrane.

In various embodiments the present invention relates to the use of a polypeptide, as herein described and defined, for the biotechnological recombinant production of chitin comprising cultivating a host cell as described above, wherein the host cell comprises a nucleic acid molecule encoding a polypeptide as defined herein above, for example a polypeptide comprising an amino acid sequence that has at least 80%, such as at least 81%, 82%, 83%, 84%, 85%, 86%, 87%, 88% or 89%, 90%, 91%, 92%, 93%, 94%, 95%, 96%, 97%, 98% or 99%, or 100% sequence identity over its entire length with the amino acid sequence set forth in SEQ ID NO:3, under conditions that allow the functional expression of the encoded polypeptide. Chitin thus produced may then be used as a substrate for the production of chitosans in vitro or by a different microorganism that expresses the necessary chitin deacetylase.

It may, in various embodiments, be advantageous to express the chitin synthase, the chitin deacetylase and, optionally, the chitinosanase, as a single polypeptide, since the spatial proximity of the chitin synthase and chitin deacetylase allows deacetylation of the formed chitin before it forms the typical chitin fibrils, which are less susceptible to deacetylation. This is a major advantage of the polypeptides described in the present invention, which are already arranged and designed to seamlessly work together in the production of chitosans.

The invention also features the respective uses of the host cells, nucleic acids and polypeptides described herein. For example, the invention further relates to the use of the isolated polypeptide for the recombinant production of chitosans (from chitin), wherein said polypeptide comprises an amino acid sequence that has at least 80%, such as at least 81%, 82%, 83%, 84%, 85%, 86%, 87%, 88% or 89%, 90%, 91%, 92%, 93%, 94%, 95%, 96%, 97%, 98% or 99%, or 100% sequence identity over its entire length with the amino acid sequence set forth in SEQ ID NO:2. As said polypeptide uses chitin as the substrate, said use may also comprise the additional use of the polypeptide of SEQ ID NO:3 or variant thereof to provide the chitin substrate.

The invention further covers to the use of the isolated polypeptide for the recombinant production of chitin, wherein said polypeptide comprises an amino acid sequence that has at least 80%, such as at least 81%, 82%, 83%, 84%, 85%, 86%, 87%, 88% or 89%, 90%, 91%, 92%, 93%, 94%, 95%, 96%, 97%, 98% or 99%, or 100% sequence identity over its entire length with the amino acid sequence set forth in SEQ ID NO:3. It is however preferred that such polypeptide additionally comprises at least the amino acid sequence of SEQ ID NO:2 or a variant thereof, to allow production of chitosan polymers.

The invention also relates to the use of the isolated polypeptide for the recombinant production of chitosan oligomers or polymers from longer chitosan polymers, wherein said polypeptide comprises an amino acid sequence that has at least 80%, such as at least 81%, 82%, 83%, 84%, 85%, 86%, 87%, 88% or 89%, 90%, 91%, 92%, 93%, 94%, 95%, 96%, 97%, 98% or 99%, or 100% sequence identity over its entire length with the amino acid sequence set forth in SEQ ID NO:1. As said polypeptide uses chitosan polymers as the substrate, said use may also comprise the additional use of the polypeptide of SEQ ID NO:2 or variant thereof and optionally also SEQ ID NO:3 or variant thereof to provide the chitosan polymers (from chitin) that serve as a substrate.

The invention also relates to the specific chitosans produced by the function of the chitinosanase of SEQ ID NO:1 or variant thereof. As this particular enzyme has a unique recognition and cleavage site, the produced chitosans differ from those produced by other chitinases, chitosanases and chitinosanases by their chemical structure. Specifically, the produced chitosans all have the sequence D(X)ₙAA, wherein D is Glucosamine (GIcN), A is *N*-Acetyl-Glucosamine (GlcNAc), X is Glucosamine (GlcN) or *N*-Acetyl-Glucosamine (GlcNAc) and n is independently 0 or an integer of from 1 to 100, preferably 0 to 50, 0 to 30, 0 to 20, 0 to 15, or 0 to 10. In various embodiments, (X)ₙ does not comprise the unit AAD, i.e. the cleavage by the chitinosanase is complete. In some embodiments, (X)ₙ may comprise additionally units AAD, which indicates that cleavage by the chitinosanase was incomplete. However, such chitosans may still serve as a substrate for the chitinosanase for another round(s) of cleavage.

All embodiments disclosed herein for the polypeptides of the invention are also applicable to all other aspects, such as the nucleic acids, host cells, methods and uses of the invention and vice versa. The invention is further illustrated, but not limited, by the following examples. All references cited herein are incorporated by reference in their entirety.

### Examples

### Materials and methods

### Cloning

*R. dicambivorans* freeze dried culture and genomic DNA was ordered from DSMZ (DSM No.: 1076509). The genomic DNA encoding for the three-domain enzyme (SEQ ID NO:14) was amplified by PCR parallelly introducing matching overhangs for subsequent cloning into a pET28a-MISTIC vector via homology directed repair using SLICE. The same method was used to clone the domains (chitinosanase domain (SEQ ID NO:12) and chitin deacetylase domain (SEQ ID NO:13) into different expression vectors (pET22b(+)::Strepll, pet22b-ST, etc.). All domains were expressed with N- and C-terminal Strep-tags and a thioredoxin domain to improve solubility.

### Expression and purification

The constructs were transformed into Escherichia coli BL21 (DE3) cells for expression. Transformed cells were inoculated in autoinduction medium for 48 h at 26°C and 120 rpm. Cells were harvested by centrifugation with 4000 x g. The pellets were resuspended in washing buffer (40 mM TEA, 400 mM NaCl, pH 8) and frozen overnight at -20°C. For lysis, the cells were thawed at room temperature, followed by addition of 3 µL benzonase (Merck KGaA, 25 U/µl), 250 µL MgCl2 (2 M) and a spatula tip of lysozyme. After incubation at room temperature for 15 min, the cells were further lysed by sonication with five pulses (15 s, 40% amplitude) using a Branson Digital Sonifier model 250-D (Emerson). Subsequently, the insoluble cell debris was removed by centrifugation with 40.000 x g at 4°C for 30 min. The proteins were purified from the supernatant by affinity chromatography using the Strep-TactinXT purification system (IBA), and eluted from the column with 20 mL elution buffer (40 mM TEA, 100 mM NaCl, 50 mM biotin, pH 8). Purified protein was concentrated with Amicon Ultra-15 centrifugal filters (Merck KGaA) and rebuffered in ammonium acetate buffer (100 mM, pH 5). Enzyme concentrations were determined via Nanodrop 2000 (Thermo Fisher Scientific).

### Chitinosanase activity assay

To determine the activity of the chitinosanase domain (SEQ ID NO:12) 0.05 mg/mL of the isolated enzyme was incubated with chitosan polymers (1 mg/mL) with three different average degrees of acetylation (DAs) (15%, 35%, and 55%) at 37°C in ammonium acetate buffer (100 mM, pH 5). Samples were taken over 24 h of the reaction and the hydrolysis reaction was repeated in triplicates for each timepoint and substrate. Part of the sample (10 µL) was mixed with 1 µL HCl (250 mM) to stop the reaction and subsequently measured by SEC-RI-MS as described previously (Hellmann et al. (2024). Fast insights into chitosan-cleaving enzymes by simultaneous analysis of polymers and oligomers through size exclusion chromatography. Scientific Reports, 14, 1-11. https://doi.org/10.1038/s41598-024-54002-2). Another part (30 µL) of the sample was filtered through centrifugal filters (3 kDa, VWR) to stop the reaction and to isolate the small oligomer products which were subsequently used to determine the subsites preferences via MS-fingerprinting.

### Chitin deacetylase activity assay

To determine the activity of the chitin deacetylase domain (SEQ ID NO:13), 0.1 mg/mL of the isolated enzyme was incubated with substrates A4 (AAAA = 4x GlcNAc) (0.25 mM) and A5 (AAAAA = 5x GlcNAc) (0.25 mM) at 37°C in 50 mM TEA buffer. Samples were taken over 24 h of the reaction and the hydrolysis reaction was repeated in triplicates for each timepoint and substrate. Part of the sample (10 µL) was mixed with 1 µL HCl (250 mM) to stop the reaction and subsequently measured by SEC-RI-MS as described previously (Hellmann et al., supra). Another part (30 µL) of the sample was filtered through centrifugal filters (3 kDa, VWR) to stop the reaction and to isolate the small oligomer products which were subsequently used to determine the subsite preferences via MS-fingerprinting.

### MS-fingerprinting

Acetylation patterns of chitosan oligomers can be analyzed using MS-fingerprinting as described previously (Cord-Landwehr et al. (2017). Quantitative Mass-Spectrometric Sequencing of Chitosan Oligomers Revealing Cleavage Sites of Chitosan Hydrolases. Analytical Chemistry, 89(5), 2893-2900. https://doi.org/10.1021/acs.analchem.6b04183). Data was analyzed using Skyline 23.1 (MacLean et al. (2010). Skyline: An open source document editor for creating and analyzing targeted proteomics experiments. Bioinformatics, 26(7), 966-968. https://doi.org/10.1093/bioinformatics/btq054), and an in-house Python script based on the module pymzML (Kösters et al. (2018). pymzML v2.0: introducing a highly compressed and seekable gzip format. Bioinformatics, 34(14), 2513-2514. https://doi.org/10.1093/bioinformatics/bty046).

### Example 1: Chitinosanase activity

DNA encoding a fragment comprising the chitinosanase domain (SEQ ID NO:12) was cloned into a pET22b(+)::Strepll vector for improved solubility and purification yields. After expression and affinity chromatography, the behavior of the enzyme on polymeric substrates was investigated. Three chitosan polymers with DAs of 15%, 35%, and 55% were digested by the isolated enzymatic domain and the reactions were monitored in real-time by continuous sampling and analysis by SEC-RI-MS.

It was found that the chitinosanase domain exhibited an increasing activity with higher DA of the substrate, demonstrated e.g. by the large number of small oligomer products in the DA55 sample after 1 h **(****Figure 1 A****),** whereas even after 48 h, there remained a high amount of polymer in the DA15 sample **(****Figure 1** **B,** peak around 300 s).

The observation from the SEC-RI chromatograms that larger polymers were continuously cleaved into smaller polymers and finally into oligomers indicates that the chitinosanase domain exhibits endo-cleaving behavior and does not degrade its substrates from the ends (van Aalten et al. (2001), Proceedings of the National Academy of Sciences 98, 8979-8984; doi:10.1073/pnas.151103798; Bußwinkel et al. (2018), International Journal of Biological Macromolecules 114, 453-461; doi:10.1016/j.ijbiomac.2018.03.070). The chitinosanase domain released only low amounts of dimeric products compared to oligomers of higher DP. Moreover, the products of the chitinosanase domain consisted mainly of highly acetylated oligomers with at least one D-unit, such as A2D1, A2D2, and A3D1 **(****Figure 1 C****).**

### Example 2: chitinosanase domain cleavage pattern

To resolve the subsite preference of the chitinosanase domain (SEQ ID NO:12) subsite specificity studies via MS-fingerprinting on polymeric substrates were performed. Samples of the chitosan digestion from example 1, specifically of DA35 and DA55, were selected to investigate the cleavage pattern. Samples of lower DA were excluded due to decreased yield of oligomers.

In all of the samples **(****Figure 2****)** the subsite -1 is predominantly A (>92 %) and the -2 subsite also shows a very high preference for A (>82 %), especially in the early timepoints (98.6% for DA55 after 1h).

However, with prolonged incubation times, D units are also accepted infrequently. Remarkably the chitinosanase exhibits a very high preference for a D unit at subsite +1 (>90%), which has not described in any GH18 chitinase. The high preference is especially pronounced in early time points and higher DAs. The -3 and +3 subsites potentially display a slight A preference (DA55, 1 h), however D units at lower DAs and longer incubations time (DA35 and DA55, 48 h) are still readily accepted. Consequently, the herein described chitinosanase domain exhibits a very specific AA|D cleavage pattern.

### Example 3: Confirming the subsite preference of the chitinosanase domain with different tetramers

Since the subsite preference analysis on polymers revealed a high specificity of the chitinosanase domain (SEQ ID NO:12) for a D unit at the +1 subsite, a detailed investigation testing the specificity of the isolated enzyme was conducted using oligomers, specifically tetramers and hexamers with defined deacetylation pattern. The enzyme itself shows clear preference for the polymers relative to oligomers as seen from reduced turnover rates and decreased activity on oligomers compared to polymers **(****Figure 1****)**.

To confirm the subsite preference of the chitinosanase domain, the isolated enzyme was incubated with five different oligomers (AAAA (A4), AADA, AAAAAA (A6), AAADAA, and AAAADA). All oligomers were digested by the chitinosanase domain over up to 24 h at 37 °C while samples were taken at several timepoints and analyzed via SEC-RI-MS.

As mentioned above, the activity of the enzyme decreases with decreasing length of the substrate. Correspondingly, it showed barely any cleavage of both tetramers, A4 and AADA **(****Figure 3A****),** although it should be noted that it was still more active on the latter. In contrast, hexamers with one D-unit which can satisfy the subsite preference (AA|D) were hydrolyzed effectively, whereas the activity on A6 was much lower in comparison. AAADAA or AAAADA were cleaved exactly as expected based on the subsite preference, resulting in the products A3 and A2D1 **(****Figure 3B****),** or A4 and A1D1 **(****Figure 3C****).** In conclusion, the digestions of defined oligomers confirmed the high specificity of the chitinosanase domain to cleave longer, high acetylated substrates only if a D-unit is bound at subsite +1.

### Example 4: Chitin deacetylase activity

The chitin deacetylase domain (SEQ ID NO:13) was cloned into a pet22b-ST vector for improved purification yields. After expression and affinity chromatography the activity of the isolated enzyme was investigated by incubation with two putative substrates, the chitin oligomers A4 and A5. Samples were taken and measured over the course of 24 h and measured at different time points using SEC-RI-MS.

Incubation of A4 and the chitin deacetylase domain resulted in a steady decrease of A4 with a parallelly occurring increase of A3D1 **(****Figure 4 A****).** After 6 h A2D2 was also observed and increased over the course of the remaining 18 h. After 24 h, A4 is completely consumed, and the largest relative fraction is A3D1. Incubation of A5 and the chitin deacetylase domain resulted in a decrease of A5 to almost 50% and a likewise mirrored increase of A4D1 to almost 50% over the course of the first 5 h **(****Figure 4 B****).** However, after the initial 5 h, both the A5 and A4D1 fractions decrease to 0% over the remaining time while A3D2 steadily increases until it reaches 100%. The activity of the chitin deacetylation domain was thus verified.

### Example 5: Chitin deacetylation pattern

After the activity of the isolated chitin deacetylase domain (SEQ ID NO:13) was confirmed, a mode of action assay was performed to determine the pattern in which the domain deacetylates chitin oligomers. Chitin oligomers A4 and A5 were again used as substrates, which were incubated with chitin deacetylase exactly as described for the activity assay and then measured after 1 h, 4 h and 24 h using MS-fingerprinting.

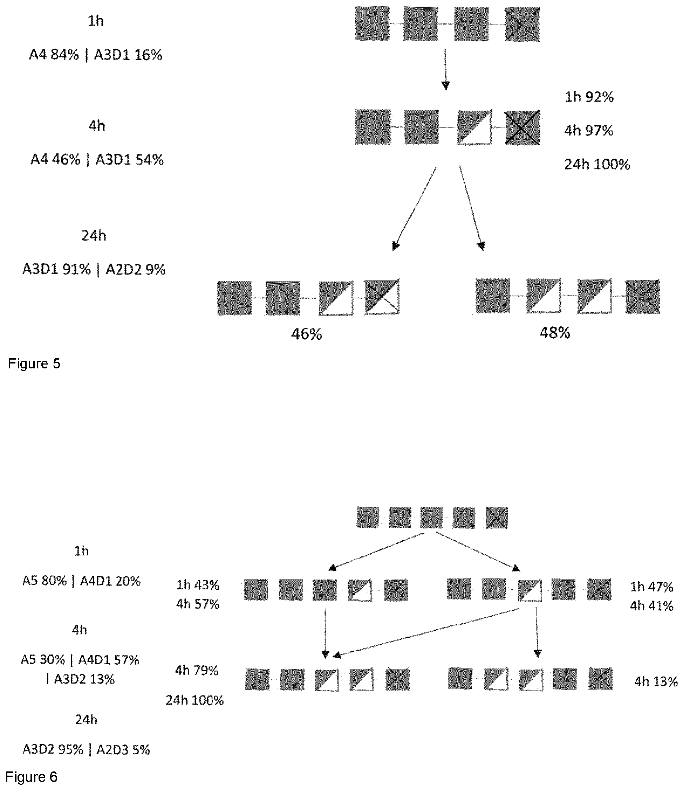

After 1 h, 84% of A4 and 16% of A3D1 is present **(****Figure 5****).** Of the A3D1, 92% is AADA. After 4 h, it is 46% A4 and 54% A3D1. Of the total A3D1, 97% is AADA. After 24 h, there is no more A4, but 91% A3D1, which is 100% AADA. This shows that the chitin deacetylase domain preferentially deacetylates the third sugar unit of chitin tetramers. In addition, 9% A2D2 is present, which is 46% AADD and 48% ADDA.

If the chitin deacetylase domain is incubated with substrate A5, 80% A5 and 20% A4D1 are present after 1 h **(****Figure 6****).** The A4D1 is 43% AAADA and 47% AADAA. After 4 h, A5 is still present at 30% and A4D1 is 57%, which is 57% AAADA and 41% AADAA. In addition, A3D2 is present at 13%, which is 79% AADDA and 13% ADDAA. After 24 h, A5 is no longer present. There is 5% A2D3 and 95% A3D2, which is 100% AADDA.

The results show that the chitin deacetylase domain follows a very specific activity pattern in which the sequence AADA is predominantly generated. Interestingly, this deacetylation pattern contains the subsite preference of the chitinosanase domain described in examples 1-3, which shows that both enzymes complement each other in their roles and activities.

## Claims

1. Isolated polypeptide comprising an amino acid sequence that has at least 80% sequence identity to the amino acid sequence set forth in SEQ ID NO:1, wherein the polypeptide preferably has chitinase activity, more preferably chitinosanase activity.

2. The isolated polypeptide according to claim 1, wherein
(1) the polypeptide has chitinase activity, preferably chitinosanase activity, and the chitinase activity, preferably chitinosanase activity, is under identical conditions at least 80% relative to the chitinosanase activity of a polypeptide consisting of the full length sequence of SEQ ID NO:4; and/or
(2) the polypeptide recognizes and cleaves a partially deacetylated chitin polymer at the motif AA | D, wherein A represents N-acetyl-D-glucosamine (GlcNAc) and D represents D-glucosamine (GlcN); and/or
(3) the polypeptide is up to 1000 amino acids in length, preferably up to 900, up to 800, up to 700, up to 600, up to 500, up to 400 or up to 300 amino acids in length; and/or
(4) the polypeptide is at least 250 amino acids in length, preferably at least 270, more preferably at least 290;
(5) the amino acid residue D at a position corresponding to position 111 and/or E at a position corresponding to position 113, using the positional numbering of SEQ ID NO:1, are invariable

3. Isolated polypeptide comprising an amino acid sequence that has at least 80% sequence identity to the amino acid sequence set forth in SEQ ID NO:2, wherein the polypeptide preferably has chitin deacetylase activity

4. The isolated polypeptide of claim 3, wherein
(1) the polypeptide has chitin deacetylase activity and the chitin deacetylase activity is under identical conditions at least 80% relative to the chitin deacetylase activity of a polypeptide consisting of the full length sequence of SEQ ID NO:4; and/or
(2) the polypeptide is up to 1000 amino acids in length, preferably up to 900, up to 800, up to 700, up to 600, up to 500, up to 400, up to 300, up to 250, or up to 200 amino acids in length; and/or
(3) the polypeptide is at least 180 amino acids in length, preferably at least 190, more preferably at least 195; and/or
(4) any one or more, preferably all, of the amino acid residues D at position 6, D at position 7, H at position 57, H at position 61, and H at position 164, using the positional numbering of SEQ ID NO:2, are invariable; and/or
(5) any one or more, preferably all, of the amino acid residues R at position 95, Y at position 98, D at position 129, D at position 132, and W at position 133, using the positional numbering of SEQ ID NO:2, are invariable.

5. Isolated polypeptide comprising an amino acid sequence that has at least 80% sequence identity to the amino acid sequence set forth in SEQ ID NO:3, wherein the polypeptide preferably has chitin synthase activity.

6. The isolated polypeptide of claim 5, wherein
(1) the chitin synthase activity is under identical conditions at least 80% relative to the chitin synthase activity of a polypeptide consisting of the full length sequence of SEQ ID NO:4; and/or
(2) the polypeptide is up to 1000 amino acids in length, preferably up to 900, up to 800, up to 700, up to 600, up to 500, up to 400, up to 300, or up to 250 amino acids in length; and/or
(3) the polypeptide is at least 200 amino acids in length, preferably at least 210, more preferably at least 220; and/or
(4) any one or more, preferably all, of the amino acid residues D at position 88, D at position 90, D at position 180, R at position 219, and W at position 220, using the positional numbering of SEQ ID NO:3, are invariable.

7. Isolated polypeptide according to any one of claims 1 to 6, comprising any two or three of
(1) an amino acid sequence that has at least 80% sequence identity to the amino acid sequence set forth in SEQ ID NO:1;
(2) an amino acid sequence that has at least 80% sequence identity to the amino acid sequence set forth in SEQ ID NO:2; and
(3) an amino acid sequence that has at least 80% sequence identity to the amino acid sequence set forth in SEQ ID NO:3,
wherein, optionally,
the polypeptide comprises (1) and (2) but not (3); the polypeptide comprises (2) and (3) but not (1);
(1) the polypeptide comprises (1) and (3) but not (2); or the polypeptide comprises (1), (2), and (3).

8. The isolated polypeptide of claim 7, wherein the polypeptide comprises the amino acid sequence of any one of SEQ ID Nos. 5-7 or a variant thereof that has at least 80% sequence identity to any one of SEQ ID Nos. 5-7.

9. The isolated polypeptide of any one of claims 1 to 8, wherein the polypeptide does not comprise the full length amino acid sequence of SEQ ID NO:4.

10. Nucleic acid molecule encoding the polypeptide of any one of claims 1 to 9, wherein the nucleic acid molecule optionally is a plasmid.

11. Host cell comprising the nucleic acid molecule according to claim 10, wherein the host cell optionally is a prokaryotic cell, preferably a bacterial cell.

12. Method for the recombinant production of chitosans comprising cultivating the host cell according to claim 11, wherein the host cell comprises a nucleic acid molecule encoding the polypeptide according to any one of claims 1 to 9, wherein the polypeptide comprises an amino acid sequence that has at least 80% sequence identity to any one or both of SEQ ID Nos. 1 and 2, under conditions that allow the functional expression of the encoded polypeptide.

13. Method for the recombinant production of chitin comprising cultivating the host cell according to claim 11, wherein the host cell comprises a nucleic acid molecule encoding the polypeptide according to any one of claims 1 to 9, wherein the polypeptide comprises an amino acid sequence that has at least 80% sequence identity to SEQ ID NO:3, under conditions that allow the functional expression of the encoded polypeptide.

14. Use of the isolated polypeptide according to any one of claims 1 to 9, wherein
(1) the polypeptide comprises an amino acid sequence that has at least 80% sequence identity to any one or both of SEQ ID Nos. 1 and 2, for the recombinant production of chitosans; or
(2) the polypeptide comprises an amino acid sequence that has at least 80% sequence identity to SEQ ID NO:3, for the recombinant production of chitin.

15. Chitosan molecule produced by the isolated polypeptide according to any one of claims 1 to 9, the host cell according to claim 11 or the method according to claim 12.
